# EUROPEAN PATENT APPLICATION

(11) **EP 3 608 414 A1**
(43) Date of publication of application: **12.02.2020**
(21) Application number: 18774601.1
(22) Date of filing: 29.03.2018
(51) Int. Cl.: C12N 15/867, C12N 15/864, C12N 15/66, C12N 7/04, A61K 48/00, A61K 9/00, A61P 3/10, A61P 37/02, A61P 29/00, A61P 19/02

(54) **VIRAL VECTOR FOR TREATING AUTOIMMUNE DISEASE AND DIABETES AND CONSTRUCTION METHOD AND APPLICATION THEREOF**

(30) Priority: 01.04.2017 CN 201710214232
(71) Applicant: Guangzhou Huazhen Pharmaceutical Co., Ltd, Guangzhou, Guangdong 510015 (CN)
(72) Inventor: ZHAO, Allan Zijian, Guangzhou, Guangdong 510015 (CN); BI, Xinyun, Guangzhou, Guangdong 510015 (CN); LI, Xiaoxi, Guangzhou, Guangdong 510015 (CN); LI, Fanghong, Guangzhou, Guangdong 510015 (CN); LIN, Yan, Guangzhou, Guangdong 510015 (CN)
(74) Representative: Lavoix
(86) International application number: PCT/CN2018/081117
(87) International publication number: WO 2018/177376

(57) **Abstract**

Provided are a viral vector for treating autoimmune disease and diabetes and a construction method and an application thereof. The viral vector is a lentiviral expression plasmid or an adeno-associated viral expression plasmid cloned with mfat-1 gene, and the mfat-1 gene is as shown in SEQ ID No.: 1.

SEQUENCE LISTING

<110> GUANGZHOU HUAZHEN PHARMACEUTICAL CO., LTD

<120> Viral vector for treating autoimmune disease and diabetes and construction
method and application thereof

<130> HP201800061EP

<160> 1

<170> PatentIn version 3.3

<210> 1
<211> 1248
<212> DNA
<213> mfat-1

<400> 1
ctagaatcgt cgacctgcag gcatggtcgc ccacagcagc gagggcctga gcgccaccgc 60

ccctgtgaca ggcggcgacg tgctggtcga cgccagagcc agcctggagg agaaggaggc 120

ccccagggac gtcaacgcca acaccaagca ggccaccacc gaggagccca gaatccagct 180

gcccaccgtg gacgccttcc ggagagccat ccccgcccac tgcttcgagc gggacctggt 240

gaagagcatc cgctacctgg tgcaggactt cgccgccctg accatcctgt acttcgccct 300

gcccgccttc gagtacttcg gcctgttcgg ctatctggtg tggaacatct tcatgggcgt 360

gttcggcttc gccctgttcg tggtgggcca cgactgcctg cacggcagct tcagcgacaa 420

ccagaacctg aacgacttca tcggccacat cgccttcagc cccctgttca gcccctactt 480

cccctggcag aagagccaca agctgcacca cgccttcacc aaccacatcg acaaggacca 540

cggccatgtg tggattcagg acaaggactg ggaggccatg cccagctgga agcggtggtt 600

caaccccatc cccttcagcg gctggctgaa gtggttcccc gtgtataccc tgtttggctt 660

ctgcgacggc agccacttct ggccttacag cagcctgttt gtgcggaaca gcgaaagagt 720

gcagtgcgtc atcagcggca tctgctgctg tgtgtgcgcc tacatcgccc tgacaatcgc 780

cggcagctac agcaactggt tctggtacta ctgggtgccc ctgagcttct tcggcctgat 840

gctggtgatc gtgacctacc tgcagcacgt ggacgacgtg gccgaagtgt acgaggccga 900

cgagtggagc tttgtgcggg gccagaccca gaccatcgac cggtactacg gcctgggcct 960

ggacaccacc atgcaccaca tcaccgacgg acacgtggcc catcactttt tcaacaagat 1020

cccccactac cacctgatcg aggccaccga gggcgtgaag aaagtgctgg agcccctgag 1080

cgacacccag tacggctaca agagccaggt gaactacgac ttcttcgccc ggttcctgtg 1140

gttcaactac aagctggact atctggtgca caagaccgcc ggcatcatgc agttccggac 1200

caccctggag gaaaaggcca aggccaagtg atgaatgcaa gctggcgt 1248

## Description

### TECHNICAL FIELD

The present invention relates to a medicament for treating diabetes and autoimmune diseases induced by immune imbalance, in particular to a viral vector for treating autoimmune disease and diabetes and construction method and application thereof.

### BACKGROUND

T lymphocyte-mediated cellular immunity is a central link in the body's immune response. The correlation between immune disorders and inflammation caused by imbalance of CD4⁺ T cell differentiation and various autoimmune diseases and neurodegenerative diseases has received sustained attention from scientists, and some mechanisms have been elucidated. CD4⁺ T cells can differentiate into Th1, Th2, Treg and Th17 under control of different transcription factors, and are in a state of dynamic equilibrium by mutual restriction of the cytokines secreted by them. Studies have shown that the imbalance between subpopulations of Th1/Th2 cells and Th17/Treg cells is closely related to various autoimmune diseases, especially type 1 diabetes (T1D), rheumatoid arthritis (RA), multiple sclerosis (MS), systematic lupus erythematosus (SLE), etc.

Type 1 diabetes, called insulin-dependent diabetes mellitus (IDDM), is a common organ-specific autoimmune disease, and is one of chronic diseases that seriously endanger health of children and adolescents. According to an assessment of American Diabetes Association, about 5% of people with diabetes are type 1 diabetes, and most of them develop symptoms in childhood or youth. In children and adolescents, T1DM is about 80-90%. In addition, there is a kind of slow-occurring latent autoimmune diabetes in adults (LADA), which is also autoimmune T1DM in etiology, but is easy to be misdiagnosed because age and clinical manifestations of these patients are similar to those of type 2 diabetes. The absolute number of patients with type 1 diabetes in China has exceeded 1 million. The whole process of type 1 diabetes is accompanied by a series of inflammations and immune disorders, mainly manifested by an attack of islet β-cells by autoreactive T cells and islet β-cell failure and necrosis caused by production of autoantibodies, leading to absolute deficiency of insulin secretion, for example, poor treatment will lead to serious complications, such as blindness, kidney failure, heart disease, amputation, etc.

Type 2 diabetes, called noninsulin-dependent diabetes mellitus (NIDDM), accounts for the vast majority of diabetes. The combination of different degrees of insulin deficiency and tissue insulin resistance is the main pathogenesis of type 2 diabetes. Once type 2 diabetes is diagnosed, islet β-cell function is progressively reduced, and hepatic glucose production and release are increased, causing elevated blood glucose.

Whether it is type 1 or type 2 diabetes, the natural course of diseases includes the following three stages: 1. Pre-hyperglycemia, which may last up to 10 years. For type 1 diabetes, there has been impairment in beta cell immunity, and its main label is an emergence of autoantibodies such as GAD or ICA. For Type 2 diabetes, there are no specific serological labels during this period, but it is often accompanied by insulin resistance syndrome such as hyperinsulinemia and abdominal obesity. Islet function shows impaired glucose tolerance (IGT) and/or impaired fasting glucose (IFG). 2. Fasting and postprandial blood glucose during hyperglycemic period meets diagnostic criteria for diabetes. 3. Chronic complications.

In recent years, the correlation between immune regulation and inflammation and diabetes has received sustained attention from scientists, and some mechanisms have been elucidated. The autoimmune response to islet antigens dominates the development and progression of type 1 diabetes. Studies have shown that excessive polarization of Th1 cells is one of the important factors leading to type 1 diabetes. For a long time, CD4⁺ effector lymphocytes have been classified as two major categories, i.e. Th1 and Th2, which are mutually restricted by their secreted cytokines and are in a state of dynamic equilibrium. When certain of β-cell autoantigens are treated by macrophages or other antigen-presenting cells, they are presented to surfaces of antigen-presenting cells together with MHC II molecules, causing releases of autoimmune signals, thereby activating Th1 cells and inhibiting releases of Th2 cells and their cytokines, which makes Th cells predominant in Th1 cell phenotype. Th1 cells further activate cytotoxic macrophages, cytotoxic T cells (CD8⁺ T cells) and natural killer cells (NK cells), and damage islet β-cells, causing inflammation of islets, resulting in a decrease or lack of insulin secretion, which eventually leads to type 1 diabetes.

Similarly, most of patients with type 2 diabetes have chronic inflammation and show insulin resistance, so type 2 diabetes is also an inflammatory disease. For example, an inflammatory mediator TNF-α may activate expressions of several proteins that inhibit an insulin signaling pathway, attenuating the body's ability to respond to insulin, and then inducing insulin resistance. Danish scientists have found that in mouse, macrophages invade diabetic pancreatic tissue in the early stages of the disease. It is published in Journal of Leukocyte Biology in January 2014 that these inflammatory cells then produce large amounts of pro-inflammatory proteins (cytokines) that act directly and damage insulin-producing β-cells in pancreas, leading to type 2 diabetes. Further studies have found that Th1 cells and CD8 T cells in acquired immunity have an effect of promoting insulin resistance, while Th2 cells and Treg cells can antagonize this effect. Therefore, controlling inflammation and regulating T cell function are also important targets for control and treatment of type 2 diabetes.

Clinically, traditional drugs such as insulin may not effectively prevent or reverse a trend of pancreatic β-cell function decline in type 2 diabetic patients. There is no effective means for prevention and treatment of type 1 diabetes, either. There are clinical studies where T cells and/or inflammatory factors are prevented from attacking islet β-cells by injecting insulin and anti-rejection drugs, but the effectiveness of the clinical studies is not supported by current findings because immunosuppressive agents usually bring many systemic side effects, including, for example, increasing the risk of tumors and infections in the body, and may also lead to insulin resistance and decreased β-cell function. In addition, a prospect of treating type 1 diabetes through islet transplantation is not optimistic. As of 2011, more than 1000 islet cell transplants have been performed in more than 50 medical institutions around the world. However, due to reasons such as poor long-term effect of islet transplantation, lack of pancreatic donors and need for long-term use of immunosuppressive agents, the number of clinical islet transplants implemented in the past few years has decreased. The monoclonal antibody Anti-CD3 may reduce a degradation of insulin secretion function and reduce an increase of insulin demand in new-onset type 1 diabetes within 2 years in the early stage of type 1 diabetes. Patients with type 2 diabetes who are younger or have more residual β-cell function can benefits more, but their clinical application is further evaluated due to side effects such as decreased immunity, fever, rash and anemia. Therefore, correct understanding of diabetes inflammation and autoimmune related pathogenesis, exploring new ways to avoid use of immunosuppressive agents, finding new intracellular factors that resist inflammation and autoimmune attacks may be more effective in treating and managing disease, and are of great significance to body and life of patients.

Rheumatoid arthritis (RA) is a chronic autoimmune disease. A recent epidemiological survey shows that the incidence of Chinese is about 0.4%, which is slightly less than the 1% of Caucasians. On this basis, there are about 4 to 5 million RA patients each year in China. There is a high prevalence of middle-aged and older women, with a male to female ratio of 1:3. An immune system of RA patients mistakenly attacks a synovial membrane of surrounding joints, causing inflammation, pain and joint damage. The cause of RA is still unclear, but in recent years, studies have widely recognized that T cell dysfunction, especially an activation of adjuvant CD4⁺ T cells, is at a center of a pathogenesis of RA. In normal human body, pro-inflammatory and anti-inflammatory factors are in an equilibrium state. For RA patients, when a certain factor initiates an immune response, Th1 and Th17 cells are activated, releasing inflammatory cytokines, causing and aggravating a progression of inflammation, while Th2 and Treg cells are inhibited. If pro-inflammatory and anti-inflammatory balance processes shift to Th1 and Th17 cells, RA may be caused. Cytokines such as IFN-γ, TNF-α, IL-2, IL-1, and IL-17 in serum and joint synovial fluid of RA patients cause occurrence and aggravation of inflammation. IL-17 synergizes with TNF-α and IL-1 in synovial inflammation, inducing a series of cytokines and chemokines, which enhance synovial inflammation. In contrast to Th1 and Th17 cells, Th2 and Treg cells are involved in anti-inflammatory effects by secreting cytokines such as IL-4 and IL-10. IL-4 secreted by Th2 cells may inhibit activities of TNF-α, IL-1, IL-6 and IL-8, exerting an anti-inflammatory effect. Treg cells may exert immunosuppressive effects in RA disease models by producing cytokines including IL-10, TGF-β, etc. The imbalance of Th cell differentiation also plays a role in cartilage destruction ^{[22]}. The cartilage is composed of cartilage tissue, which is composed of chondrocytes, stroma and collagen fibers, and surrounding perichondrium. A balance is formed between bone formation and bone resorption during normal physiological processes, and inflammation is closely linked to bone erosion. In recent years, it has been found that nuclear factor-κB receptor activating factor (RANK) binds to its receptor RANKL mediating differentiation and maturation of osteoclasts. OPG may bind to RANK, and block binding of RANKL to RANK, thus inhibit differentiation and activation of osteoclasts and induce apoptosis of mature osteoclasts. IL-17 may up-regulate expressions of RANKL and its receptors, thereby disrupting a balance of RANKL/OPG in synovial fluid and aggravating bone destruction. IL-4 and IL-10 produced by Th2 cells inhibit the differentiation of osteoclasts. Treg cells acting as Th17 antagonists may directly inhibit osteoclast formation, and Treg cells may up-regulate OPG expression and down-regulate RANKL through self-secreting IL-10, thereby inhibiting bone destruction.

There is currently no method for curing RA. The usual drugs and physical methods may only treat symptoms in RA patients, reduce inflammation and pain, and delay RA progression. The first-line chemical drugs currently used in treatment of RA are mainly nonsteroidal anti-inflammatory drug (NSAID), including gold compounds, D-penicillamine, antimalarials, sulfasalazine, methotrexate, etc., which reduce damage to the joints primarily by suppressing the immune system. The biologic drugs, such as infliximab, etanercept, anakinra for the treatment of RA mainly target TNF-α and IL-1 to antagonize the pro-inflammatory effects of TNFα and IL-1. These genetically engineered biopharmaceuticals may reduce acute inflammatory responses and reduce swelling and pain in patients. However, 30%-40% of patients have no response to TNF-α antagonists, and some patients develop antibodies to a chimeric antibody, causing efficacy losing of the chimeric antibody. In addition, TNF-α is an important cytokine in the normal immune system, and blocking it may cause serious side effects and increase the risk of infection. Therefore, for the current anti-rheumatic drugs, short-term and long-term tolerance and safety concerns have been raised. So it is necessary to further develop new drugs that can cure the symptoms, block autoimmunity, and rebalance immune regulation.

Multiple sclerosis (MS) is the most common central demyelinating disease. In the acute active phase of this disease, a central nervous system has multiple inflammatory demyelinating plaques, and old lesions form calcified plaques due to glial fibrosis. The disease is characterized by multiple lesions, remission, and recurrence, and occurs in an optic nerve, a spinal cord, and a brain stem, and is more common in young and middle-aged women, and more common in women than in men. In human MS pathology studies, it was found that the pathology of human MS is similar to that of mouse experimental allergical encephalomyelitis (EAE). The mechanism of MS is that MB cells-specific Th cells penetrate blood-brain barrier and enter the central nervous system, and contact MBP to develop an immune response, stimulate the inflammatory response, and produce infiltration of inflammatory factors and chemokines, resulting in destruction of a spinal sheath, so as to cause symptoms such as visual impairment and muscle weakness in the patient ^{[25]}. MS mainly involves Th1 and Th17 responses. IL-2, IFN-γ and IL-17 are increased significantly in the lesion area. In a recovery period, Th1 type cytokines decline as IL-4, IL-10 and TGF-β increasing. EAE development may be prevented by feeding specific Th2 cells ^{[27]}. A similar situation is also found in MS clinical studies. Therefore, blocking the imbalance of Th cell differentiation will also play a crucial role in the treatment of MS.

There is no method for completely curing RA. Currently used treatments focus on suppressing the inflammatory response caused by immunity. At present, FDA has approved six kinds of drugs for the treatment of MS, comprising immunomodulators (Interferon-β1a, subcutaneous and intramuscular injections, Interferon-β1b subcutaneous injections, Glatiramer acetate), immunosuppressive agents (mitoxantrone) and monoclonal antibodies (Natalizumab). Interferon-β has an advantage of high safety, but may only reduce a number of recurrences by 30%, and it is expensive and not suitable for widespread promotion. Mitoxantrone is an anticancer drug used primarily to treat leukemia, breast cancer, lymphoma and liver cancer, etc. Mitoxantrone may inhibit proliferations of T cells, B cells and macrophages, exerting immunosuppressive effects. Mitoxantrone has serious side effect to myocardial toxicity. Myocardial toxicity is caused when the cumulative dose of mitoxantrone is greater than 140 mg/m². Mitoxantrone can be used for up to three years at a rate of 12 mg/m² every three months, so it is too short-lived with respect to average 30-year course of MS ^{[30]}. In addition to mitoxantrone, other immunosuppressive agents such as azathioprine, cyclophosphamide, methotrexate, mycophenolate mofetil and tacrolimus have short-term immunosuppressive effects, but have evident side effects. For example, cyclophosphamide may cause hemorrhagic cystitis, methotrexate may still cause liver toxicity at low dose, and mycophenolate Mofetil, tacrolimus and azathioprine have myelosuppressive effects. Natalizumab neutralizes an adhesion molecule such as α-4integrin on a surface of lymphocytes, preventing it from binding to ligands (vascular cell adhesion molecule-1,VCAM-1) on vascular endothelial cells and stromal cells, inhibiting lymphocytes from penetrating a vascular endothelium and reaching tissues, so as to reduce autoimmune inflammation in brain tissue, and inevitably affect overall immune responses, infect JC virus and increase a risk of progressive multifocal leukoencephalopathy (PML). Natalizumab also has severe liver toxicity. MS is a prolonged and refractory chronic autoimmune disease. Therefore, in the development of therapeutic drugs, safety and convenience of long-term use need to be considered, in addition to efficacy. The best way to solve these problems is undoubtedly to effectively change the autoimmune attack caused by immune disorders.

SLE is a chronic autoimmune disease. The cause of the disease is unknown. The patient's immune system produces autoantibodies to attack cells and tissues of the body, causing inflammation and tissue damage. Common symptoms include arthritis, fevers, chest pains, hair losses, mouth ulcers, swollen lymph glands, fatigues, and facial rashes, etc. The pathogenesis of SLE is unknown. Studies have shown that T-cell abnormalities play an important role in the pathogenesis and development process of SLE. Under the interaction of genetic factors, environmental factors, estrogen levels and other factors, autoreactive Th-cells in SLE are activated by autoantigens and undergo clonal expansion, and produce cytokines. In MRLP lpr lupus mouse, a secretion of IFN-γ increased, and a proportion of Th1/Th2 cells increased. In patients with type IV diffuse proliferative lupus nephritis (DPLN), Th1/Th2 cells in peripheral blood and kidney are significantly elevated, which is positively correlated with a degree of renal damage, suggesting that Th1 cells also play an important role in the pathogenesis of SLE. At the same time, in patients with SLE, the number and function of Treg cells decrease and B cells hyperplasia, a large number of autoantibodies are produced, and combined with the corresponding autoantigens in the body to form corresponding immune complexes, which are deposited in skin, joints, small blood vessels, small kidneys ball and other parts. Acute and chronic inflammations and tissue necrosis (such as lupus nephritis) are caused with a participation of complements, or antibodies directly interact with tissue cell antigens to cause cell destruction, resulting in multiple system damage.

Up to now, there is no method for completely curing SLE. Currently used therapeutic drugs include non-steroidal anti-inflammatory drugs (NSAIDs), corticosteroids, immunosuppressive agents, hydroxychloroquine, and methotrexate. The non-steroidal anti-inflammatory drugs have common side effects including gastrointestinal discomforts, heartburn, diarrheas, and causing body to retain water. The corticosteroids may quickly suppress inflammation. Because of their high potency, the lowest dose is usually used to achieve maximum efficacy. The corticosteroids have short-term side effects including edemas, increased appetites, and weight gains, and long-term side effects including osteopenia (skeletal loosening), high blood pressure, high cholesterol, high blood sugar (diabetes), arterial damages, infections, and cataracts. The immunosuppressive agents have common side effects including nauseas, vomiting, hair losses, bladder problems, difficulty in conception, increased risk of cancer and infection. In patients with systemic lupus erythematosus, on the one hand, the disease may recur after stopping administering of drugs, and on the other hand, the longer the duration of administering, the higher the risk of side effects. Therefore, a research direction of completely curing SLE is to improve an imbalance of Th cell differentiation in SLE, so as to alleviate and reverse autoimmune attacks.

Polyunsaturated fatty acids (PUFAs) are linear fatty acids having 18-22 carbon atoms and containing 2-3 hydrogen bonds, and usually comprise two types, one is ω-3 PUFAs having a most distal double bond from a carboxyl group at third last carbon atom and the other is ω-6 PUFAs having a most distal double bond from a carboxyl group at sixth carbon atom. Both of ω-6 and ω-3 PUFAs are synthesized from saturated fatty acid precursors with different desaturases. Vertebrates and mammals lack specific desaturases, and therefore may not synthesize ω-6 and ω-3 in vivo, but can only ingest them from diets.

ω-3 PUFAs are extremely important for maintaining normal physiological functions, and are key components of cellular lipid membranes. Numerous studies have shown that maintaining a proportional balance of ω-6/ω-3 PUFAs plays an important role in health. Two recent large-scale clinical studies have shown that long-term intake of ω-3 PUFAs is inversely proportional to an incidence of type 1 diabetes. A control study based on 2,000 Norwegian children shows that taking cod liver oil rich in ω-3 PUFAs from the first year after birth may reduce a risk of type 1 diabetes in children. A follow-up survey of diabetes autoimmunity study in the young (DAISY) shows that a risk of autoimmune inflammation in islets may be reduced after intake of ω-3 PUFAs in children with a genetic risk of type 1 diabetes. In addition, free fatty acids such as palmitic acids inhibit insulin secretion in vitro, which may be reversed by ω-3 PUFAs. Glucose-stimulated insulin secretion may be enhanced by dietary supplementation with ω-3 PUFAs. In addition, in recent years, a number of clinical studies have suggested that ω-3 PUFAs have functions such as inhibiting inflammation and preventing and alleviating autoimmunity, and have protective effects on pathogenesis and pathological developments of RA, MS and SLE. However, clinical attempts to treat diabetes and control body weight using fish oil capsules containing ω-3 PUFAs have mostly failed. One reason is that a content of ω-6 PUFAs in ingested food is too high, and it is difficult to balance a ratio of ω-6/ω-3 PUFAs by simply supplementing the fish oil capsules. Therefore, the present invention proposes a viral vector carrying fat-1 gene encoding ω-3 fatty acid desaturase, which promotes a conversion of ω-6 to ω-3 in vivo, so as to balance the ratio of ω-6/ω-3 PUFAs.

A protein product of the fat-1 gene derived from C. elegans is ω-3 unsaturated fatty acid desaturase, which is desaturated with ω-6 PUFAs as a substrate to form the corresponding ω-3 PUFAs, thereby reducing the content of ω-6 PUFAs and change the ratio of ω-6/ω-3 PUFAs while significantly increasing the content of endogenous ω-3 PUFAs in animals. In order to make the fat-1 gene better expressed in mammals, cDNA encoding the fat-1 gene is mammalianized and is named mfat-1. Islet β-cells of mfat-1 transgenic mouse can significantly resist cytokine-induced anti-apoptotic effects, and ω-3 PUFAs can promote insulin secretion.

### SUMMARY

A first technical problem to be solved by the present invention is to provide a viral vector, which is safe and non-toxic, has therapeutic activity, and can meet clinical needs.

A second technical problem to be solved by the present invention is to provide a method for constructing the above viral vector.

A third technical problem to be solved by the present invention is to provide use of the above viral vector in the manufacture of a medicament for treating diabetes or autoimmune related diseases.

In order to solve the above technical problem, the present invention provides a viral vector, which is a lentiviral expression plasmid cloned with mfat-1 gene or an adeno-associated viral expression plasmid cloned with mfat-1 gene, and the mfat-1 gene is as shown in SEQ ID No.: 1.

In the viral vector, the lentiviral expression plasmid is pLJM1-CMV-hPGK-EGFP plasmid, pLJM1-CMV-hPGK-mkate2 plasmid, pLenti-CMV-MCS-GFP-SV-puro plasmid, FUGW, pLenti-puro, pLenti-MP2 or pLenti plasmid; and the adeno-associated viral expression plasmid is pEMBL-AAV-D(+)-CMV-eGFP-SV40 plasmid, AAV GFP plasmid, AAV1 plasmid, AAV2 plasmid, rAAV2 plasmid, AAV5 plasmid, AAV8 plasmid, AAV9 plasmid or pAV-FH AAV plasmid.

The present invention provides a method for constructing the viral vector, comprising obtaining the viral vector by cloning the mfat-1 gene into a lentiviral expression plasmid or an adeno-associated viral expression plasmid.

The method for constructing the viral vector comprises steps of:
(1) designing primers by using mfat-1 gene sequence as a template, and carrying out PCR amplification of the mfat-1 gene sequence with the primers, to obtain a PCR amplification product which is a mfat-1 gene sequence with NheI and EcoRI restriction sites at both ends; wherein the primers are as follows:
   mfat-1-F: 5'-TATTAAGCTAGCATGGTCGCCCACAGCA-3';
   mfat-1-R: 5'-CAACCGGAATTCTCATCACTTGGCCT-3';
(2) electrophoresing the PCR amplification product obtained in the step (1) to obtain a gel, then cutting, recovering and purifying the gel to obtain a purified PCR amplification product, digesting the purified PCR amplification product with restriction enzymes NheI and EcoR to obtain a DNA fragment, and digesting an empty lentiviral expression plasmid or adeno-associated viral expression plasmid with restriction enzymes NheI and EcoR to obtain a digested empty shuttle plasmid;
(3) ligating the DNA fragment obtained in step (2) to the digested empty shuttle plasmid to obtain the viral vector.

In the method for constructing the viral vector, a reaction system of the PCR amplification in the step (1) comprises:
5×PrimeSTAR® Buffer (Mg2+ plus), 10 µl;
dNTP Mixture, each dNTP 2.5 mM, 4 µl;
template DNA, 20-200ng;
upstream primer mfat-1-F, 10 µM, 1 µl;
downstream primer mfat-1-R , 10 µM, 1 µl;
PrimeSTAR® HS DNA Polymerase, 2.5 U/µl, 0.5 µl;
adding sterile ultrapure water to 50µL.

Preferably, the template DNA is 100 ng.

In the method of constructing the viral vector, a reaction procedure of the PCR amplification in the step (1) is as follows: denaturation at 98 °C for 5 min; and holding at 98 °C for 10 s, and holding at 60 °C for 15 s, holding at 72 °C for 2 min, a total of 30 cycles; finally extension at 72 °C for 10 min.

The present invention provides a recombinant viral vector, which is constructed by the viral vector.

The present invention provides a viral particle, which is obtained by transforming the viral vector into a cell.

Preferably, the viral particle is obtained by transforming the viral vector with lipofection into 293FT cell, and packaging and amplifying.

Preferably, the viral particle is administered by intravenous injection.

The present invention provides use of the viral vector or the recombinant viral vector or the viral particle in the manufacture of a medicament for treating diabetes or treating autoimmune related diseases.

In the use, the autoimmune related diseases are autoimmune diseases induced by imbalance of Th cell differentiation and imbalance of its secreted cytokines.

In the use, the diabetes is autoimmune type 1 diabetes.

In the use, the autoimmune disease is selected from type 1 diabetes (T1D), multiple sclerosis (MS), rheumatoid arthritis (RA) and systemic lupus erythematosus (SLE). The present invention provides a medicament, which comprises the viral vector or the recombinant viral vector or the viral particle, and a pharmaceutically acceptable carrier thereof.

Preferably, the medicament is prepared into a clinically acceptable dosage form with a conventional process by mixing the viral vector or the recombinant viral vector or the viral particle with conventional excipients.

The lentiviral expression plasmid is pLJM1-CMV-hPGK-EGFP plasmid (purchased from Addgene (Plasmid #19319)) and pLJM1-CMV-hPGK-mkate2 plasmid (purchased from addgene).

Wherein, the adeno-associated viral expression plasmid is pEMBL-AAV-D(+)-CMV-eGFP-SV40 plasmid (purchased from addgene).

In the present invention, a gene therapy method is adopted, wherein the mfat-1 gene is introduced into a self-expression non-obese diabetic model mouse (NOD mouse) by using the viral particle of the present invention. Compared with a control group, mfat-1 lentiviral particles can significantly reduce blood glucose levels of the NOD mouse, and increase serum insulin levels, having a significant therapeutic effect. Possible mechanisms for the treatment of diabetes and autoimmunity using mfat-1 gene therapy include: using high levels of ω-6 PUFAs in the body as a substrate, mfat-1 converted it into ω-3 PUFAs, so that a ratio of ω-6/ω-3 is balanced and tends to 1:1. These endogenous ω-3 PUFAs transformed from ω-6 PUFAs promote a differentiation of Th cells into Th2 and antagonize an effect of Th1 cell activation by ω-6 PUFAs, reduce secretion levels of pro-inflammatory factors such as IFN-γ, IL-6, TNF-α, and IL-17, alleviate the Th1 cell-mediated inflammatory response, inhibit infiltrations of CD8⁺ CTL cells and macrophages in islets, and alleviate occurrences of islet inflammation and peripancreatitis. Since there lack an unsaturated fatty acid dehydrogenase in mammals, it is necessary to introduce the mfat-1 gene into the body using a lentiviral vector or an adeno-associated viral vector.

The above viral vector is administered by intravenous injection.

The present invention has the following beneficial effects: when compared with the prior art, the viral vector of the present invention can be used for preparing a biological drug for treating type 1 diabetes and related autoimmune diseases, for example, a viral particle obtained by transfecting a viral vector into a cell is a safe and non-toxic novel biological drug having anti-inflammatory activity. When using the lentivirus as a vector to high express polyunsaturated fatty acid dehydrogenase by gene recombination technology, anti-autoactive inflammatory activity is enhanced and can meet the needs of use. The preparation method is simple and easy to operate, thus has a good application prospect.

### BRIEF DESCRIPTION OF THE DRAWINGS

Fig. 1 is a 1% agarose gel electrophoresis pattern of pLJM1-mfat-1-EGFP plasmid after digestion with NheI and EcoRI;
Fig. 2 is a graph showing results of differentiations of human peripheral blood CD4 T-cells and changes in cytokines thereof, after treating peripheral blood mononuclear cells of normal humans (Fig. 2A-E) and type 1 diabetic patients (Fig. 2F-J) in vitro for 24 h by using lentiviral particles in Example 2 of the present invention;
Fig. 3 is a graph showing results of changes in peripheral blood CD4 T-cell transcription factors after treating peripheral blood mononuclear cells of patients with type 1 diabetes in vitro for 24 h according to Example 2 of the present invention;
Fig. 4 is a graph showing results of changes in incidences of diabetes in NOD mice after administrating lentivirus for 9 weeks in Example 3 of the present invention.
Fig. 5 is a graph showing results of blood glucose changes in NOD mice after administrating lentivirus in Example 3 of the present invention;
Fig. 6 is a graph showing results of insulin secretion in NOD mice after administrating lentivirus for 9 weeks in Example 3 of the present invention;
Fig. 7 is a graph showing results of changes in the development of islet inflammatory infiltration in NOD mice after administrating lentivirus for 9 weeks of in Example 3 of the present invention;
Fig. 8 is a graph showing results of differentiation of CD4 cells in NOD mice after administrating lentivirus for 9 weeks in Example of the present invention.

### DETAILED DESCRIPTION

The invention can be better understood in light of the following examples. However, it is to be easily understood by those skilled in the art that the description of examples is only intended to illustrate the invention and should not be construed as limiting the invention as described in claims. In the following examples, DH5α E. coli is supplied by TAKARA; LB medium is supplied by Sigma; 293FT cell line is supplied by ATCC; growth medium DMEM is supplied by GIBCO; FBS fetal bovine serum is supplied by GIBCO; OPTI-MEM is supplied by GIBCO; Lipofectamine 2000 transfection reagent is supplied by Invitrogen-Thermo Fisher Scientific; NOD mouse is supplied by The Jackson Laboratory. A centrifuge is FRESCO17 high-speed refrigerated centrifuge manufactured by American Thermo Company; an electrophoresis apparatus is PowerPac™ and Mini-Sub cell GT manufactured by American BIO-RAD company; a multiImager is ChemiDoc™ XRS+ System manufactured by American BIO-RAD company.

### Example 1: Construction of a viral vector cloned with mfat-1 lentiviral expression plasmid.

Firstly, a mfat-1 gene as shown in SEQ ID No.: 1, provided by Kingsray, was synthesized, and then was subcloned to pLJMI-CMV-hPGK-EGFP plasmid (addgene, Plasmid #19319) by NheI and EcoRI restriction sites, to obtain pLJM1-CMV-hPGK-EGFP-mfat-1 (PLJM1-mfat-1) expression plasmid. The detailed construction process is as follows:

### 1) PCR amplification of mfat-1 region

Primers were designed by using the synthesized mfat-1 gene sequence as a template. PCR amplification of the mfat-1 gene sequence was carried out with the primers to obtain a PCR amplification product which is a mfat-1 gene sequence with NheI and EcoRI restriction sites at both ends, wherein the primers are as follows:
upstream primer mfat-1-F: 5'-TATTAAGCTAGCATGGTCGCCCACAGCA-3';
downstream primer mfat-1-R: 5'-CAACCGGAATTCTCATCACTTGGCCT-3'.

A system of PCR amplification reaction is shown in Table 1.

**Table 1**

| System of PCR amplification reaction | |
|---|---|
| 5×PrimeSTAR® Buffer (Mg2+ plus) | 10 µl |
| dNTP Mixture (each dNTP 2.5 mM) | 4 µl |
| template DNA 100 ng | |
| upstream primer (10 µM) | 1 µl |
| downstream primer (10 µM) | 1 µl |
| PrimeSTAR® HS DNA Polymerase (2.5 U/µl) | 0.5 µl |
| adding sterile ultrapure water to 50µL | |

A procedure of the PCR amplification reaction is as follows: denaturation at 98 °C for 5 min; and holding at 98 °C for 10 s, and holding at 60 °C for 15 s, holding at 72 °C for 2 min, a total of 30 cycles; finally extension at 72 °C for 10 min.

### 2) Digestion

The PCR amplification product obtained in the above step was subjected to 1.5% agarose gel electrophoresis to recover a target DNA fragment. An Agarose Gel DNA Extraction Kit was used to obtain a purified target DNA fragment, which was digested with restriction enzymes NheI and EcoR to obtain a digested target DNA fragment. A pLJM1-EGFP empty lentiviral expression plasmid was digested with restriction enzymes NheI and EcoR to obtain a digested pLJM1-EGFP plasmid. A reaction system of digestion is shown in Table 2:

**Table 2**

| System of digestion reaction | |
|---|---|
| plasmid or target DNA fragment | 1 µg |
| 10×buffer | 2 µl |
| endonuclease NheI | 1 µl |
| endonuclease EcoRI | 1 µl |
| adding sterile ultrapure water to 20µL | |

Conditions of digestion reaction: 37 °C water bath for 2 h. The above digested DNA fragment was subjected to 1% agarose gel electrophoresis to separate a target DNA fragment, and a purified plasmid or target DNA fragment was obtained using an Agarose Gel DNA Extraction Kit.

### 3) Ligation

The digested target DNA fragment was ligated to the digested pLJM1-EGFP plasmid.

**Table 3**

| System of ligation reaction | |
|---|---|
| digested pLJM1-EGFP plasmid | 45 ng |
| digested target DNA fragment | 60 ng |
| 10×buffer | 2 µl |
| ligase (350 units/µl) | 1 µl |
| adding sterile ultrapure water to 20µL | |

Conditions of ligation reaction: ligating at room temperature (16 °C) overnight, i.e. 12 h, to obtain a PLJM1-mfat-1 vector, i.e. a ligated product.
The example also provides a viral particle, which is prepared by transforming the above viral vector into a cell. Detailed steps are as follows:

### 1. A competent bacteria used for transformation is DH5α E. coli, prepared by CaCl₂ method. The preparation method and subsequent transformation operations refer to the second edition of Molecular Cloning, as follows:

### A. Preparation of a competence:

i. 1 ml saturated bacterial solution was added to 100 ml LB, and then shaken for 2-3 h at 37 °C, 280 rpm, until an optical density OD600 reflecting the bacterial density was between 0.4 and 0.6;
ii. the solution obtained in step i was transferred to ice-cold 50 ml polypropylene tube and then placed in an ice bath for 10 min;
iii. the solution obtained in step ii was centrifuged for 10 min at 4 °C, 1000 g, to obtain a supernatant and a precipitate;
iv. the supernatant was discard, the tube was inverted for 1 min to make the supernatant drained, and the precipitate was resuspended by adding 10 ml ice-cold 0.1 mol/l CaCl₂, and placed in an ice bath for 30 min;
v. the solution obtained in step iv was centrifuged for 10min at 4 °C, 1000g, to obtain a supernatant and a precipitate;
vi. the supernatant was discard, and the precipitate was resuspended by adding 2 ml ice-cold 0.1 mol/l CaCl₂ , and then was well mixed with 0.5 ml 75% sterilized glycerol (prepared with 0.1 mol/l CaCl₂ ) to obtain a mixture. The mixture was dispensed into a 1.5 ml centrifuge tube at 100 µl/tube, and can be stored in a -80 °C refrigerator for half a year;
vii. a conversion efficiency was identified.

### B. Conversion of the ligated product:

i. 1 tube of competent cells was thawed on ice, 3 µl ligated product was added thereto, mixed well, and then placed in ice bath for 30 min;
ii. the solution obtained in step i was left to stand in 42 °C water bath for 90 sec, and then transferred quickly to ice for ice bath for 1-2 min;
iii. the solution obtained in step ii was mixed with 900 µl LB medium, then placed in 37 °C water bath for 10 min;
iv. the bacteria were amplified for 45 min at 37 °C, 210 rpm, to obtain transformed bacteria;
v. 100 µl of the above transformed bacteria were coated on an agar plate containing 100 µg/ml ampicillin;
vi. the plate was inverted and incubated at 37 °C for 16-20 h.

### C. Positive clone screening (digestion identification, sequencing identification)

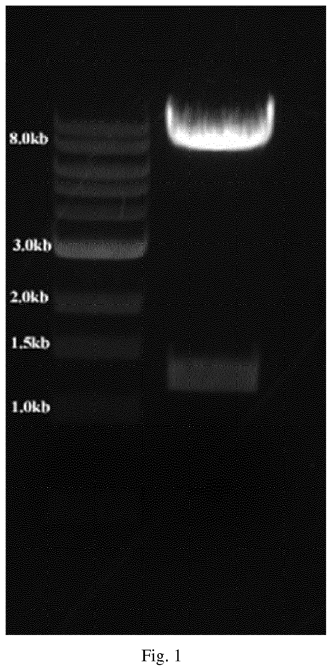

The positive clone was identified by 1% agarose gel electrophoresis, and the results are shown in Fig. 1, which indicates that the viral vector PLJM1-mfat-1 was successfully constructed.

### 2. Packaging, amplification and purification of the lenti-mfat-1 lentiviral particle.

A packaging cell of lentivirus is 293FT cell strain, and its growth medium is DMEM (containing 10% FBS). Adherent cells were proliferated in the medium to form monolayer cells.

### (1) Amplification of virus:

1) 293FT cells in logarithmic growth phase were digested with trypsin, and adjusted to a cell density of 1×10⁵ cells/ml using DMEM high glucose medium (supplied by GIBCO) containing 10% FBS fetal bovine serum (provided by GIBCO), and then inoculated in a six-well plate, and cultured at 37 °C in a 5% CO₂ incubator. After 24 h, the cell density can reach 0.8×10⁶ cells/ml and can be used for transfection. State of the cells is critical for packaging of virus, therefore the cells need to be in good state and have fewer passages.
2) The cell culture medium was replaced with fresh complete medium 1 h before transfection.
3) In one 1.5 ml EP tube, a plasmid reagent, including 1.5 µg PLJM1-mfat-1 vector, 1.125 µg psPAX2 vector Addgene (Plasmid #12260), 0.375 µg pMD2.G vector Addgene (Plasmid #12259), was added, and then mixed well with 100 µl OPTI-MEM and incubated for 5 min at room temperature (20 °C - 30 °C).
4) In another 1.5ml EP tube, 100µl OPTI-MEM was firstly added, and then 3µl Lipofectamine2000 transfection reagent was added, mixed gently, and incubated for 5 min at room temperature (20 °C -30 °C).
5) The incubated plasmid reagent (mixture of three kinds of plasmids PLJM1-mfat-1, psPAX2 and pMD2.G) obtained in step 3) was mixed gently with the diluted Lipofectamine 2000 transfection reagent obtained in step 4), incubated for 15 min at room temperature (20°C - 30°C) to form a transfection complex of the plasmid reagent with the Lipofectamine 2000 transfection reagent.
6) 200 µl reagent obtained in step 5) (i.e. the transfection complex of PLJM1-mfat-1, psPAX2 and pMD2.G and Lipofectamine2000 transfection reagent, obtained in step 5)) was dropped into the culture medium containing 293FT cells in obtained step 1), gently shaken and incubated at 37 °C in a 5% CO 2 cell incubator.

### (2) Purification of virus:

1) After the 293FT cell culture medium was cultured for 24 h in the above steps, the medium containing the transfection complex was discarded, and 2 ml fresh complete medium was added to continue the culture.
2) After continuing the culture for 48h and 72h respectively, the 293FT cell culture mediums were centrifuged to collect supernatants respectively. The supernatants collected at 48h and 72h were mixed, centrifuged at 1250 rpm for 5 min to remove the 293FT cells, and filtered with 0.45 µm filter to obtain the Lenti-mfat-1 lentiviral particle. The viral particle may be stored at 4 °C for a short time (<3d), and may be stored at -80 °C for a long time, and was separate packed to avoid repeated freezing and thawing.

### (2) Determination of titer of virus

The titer of virus was determined by TCID50 method. The amplification, purification and determination of titer of control naked virus were carried out with the same methods described as above.

### Example 2: Regulation of ω-3 PUFAs on peripheral blood CD4 T-cells in patients with type 1 diabetes

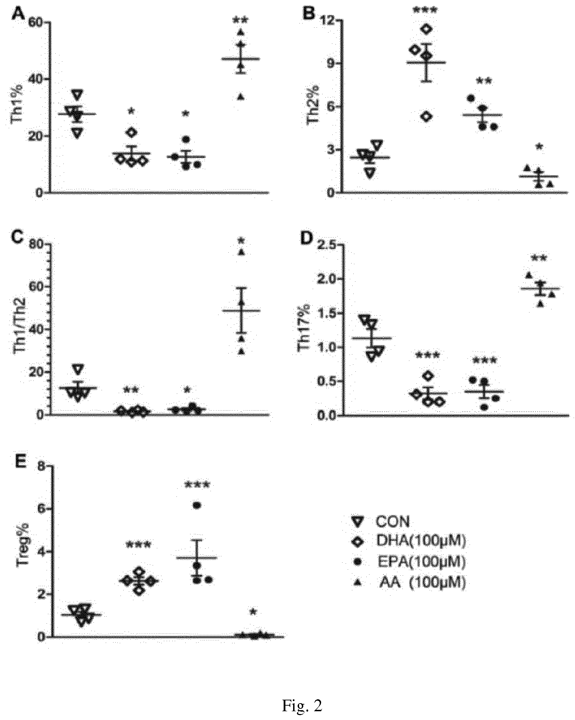

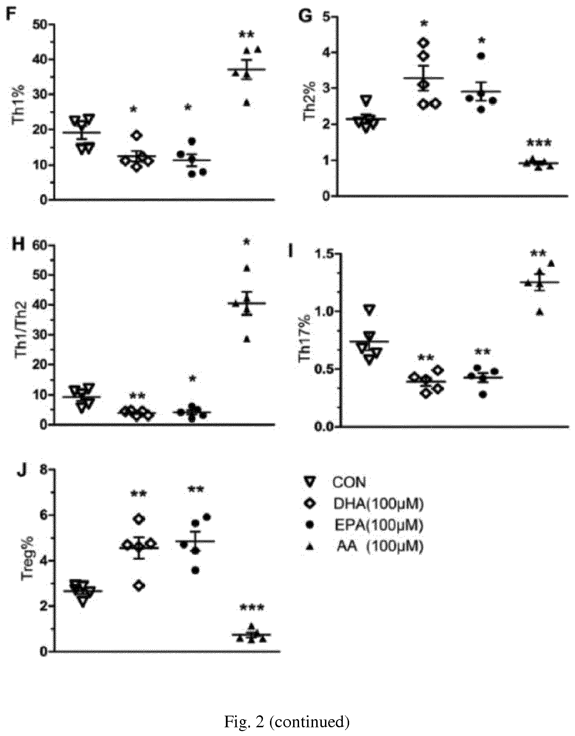

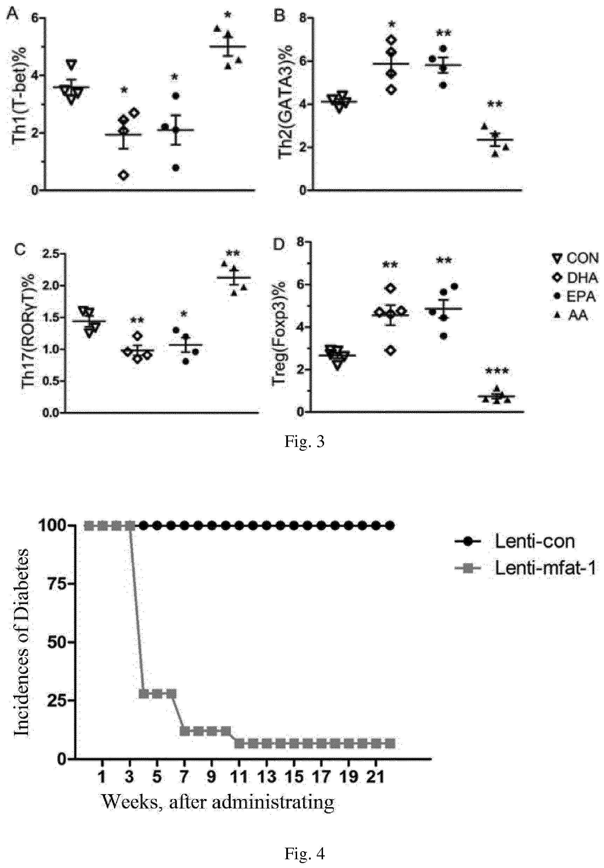

(1) 5 ml peripheral blood was extracted from patients and normal humans separately, diluted twice with PBS of pH 7.2, and then mixed with lymphocyte separation solution Lymphoprep (Axis-Shield, Norway), centrifuged at 3500 rpm for 20 min, then the lymphocytes were taken out and cultured in RPMI1640 medium (purchased from Gibco) to obtain the cultured lymphocytes. The above cultured lymphocytes were treated in vitro with DHA (docosahexaenoic acid) at a final concentration of 100 µM, EPA (eicosapentaenoic acid) at a final concentration of 100 µM, and AA (arachidonic acid) at a final concentration of 100 µM for 24 h, respectively. After treating, changes in intracellular cytokines of CD4 T-cells in the above lymphocytes were detected. (The above DHA, EPA and AA are all purchased from Sigma).
(2) labeling of intracellular cytokines of CD4 T-cells: The lymphocytes (number up to 1×10⁵) from patients and normal humans in the above steps were placed to different marked tubes. Labeled fluorescent monoclonal antibodies CD3 (0.1µg) and CD8 (0.1µg) were added into each tube. Then 1ml paraformaldehyde having a concentration of 4% by weight was added into each tube, mixed well and incubated at room temperature (20°C-30°C) for 20 min. 1ml intraprep permeabilization reagent, i.e. 0.5% saponin, was added, incubated at room temperature (20°C-30°C) for 30 min, then centrifuged for 5 min at 300 g, 4 °C to obtain a supernatant and a precipitate. The supernatant was removed, and 80µl 0.5% saponin was added to re-suspend the precipitate to obtain suspensions. The obtained suspensions were labeled respectively with IFN-γ fluorescent monoclonal antibody (0.1 µg), IL-17 fluorescent monoclonal antibody (0.1 µg), IL-4 fluorescent monoclonal antibody (0.1 µg), and Foxp3 fluorescent monoclonal antibody (0.1 µg), and incubated in dark place at 4°C for 1 h (for the suspension labeled with Foxp3 fluorescent monoclonal antibody, incubation and rupture took 8 h). The labeled cells were washed three times with 0.5% saponin, centrifuged for 5min at 300g, 4°C to obtain a supernatant and a precipitate. The supernatant was removed, and the precipitate was fixed with paraformaldehyde having a mass concentration of 1%. After 24 h, percentages of Th1, Th2, Th17 and Treg cells were analyzed with a flow cytometer Accuri-C6. The antibodies and instruments used in the above steps were all purchased from BD Bioscience.
(3) Labeling of intracellular transcription factors of CD4T cells: The lymphocytes (number up to 1×10⁵) from patients and normal humans in the steps 1) were placed to different marked tubes. Labeled fluorescent monoclonal antibodies CD3 (0.1µg) and CD8 (0.1µg) are added into each tube. 1ml 4% paraformaldehyde was then added into each tube, mixed well and incubated at room temperature for 20 min. 1ml intraprep permeabilization reagent, i.e. 0.5% saponin, was added, incubated at room temperature for 30 min, then centrifuged for 5 min at 300 g, 4 °C to obtain a supernatant and a precipitate. The supernatant was removed, and the precipitate was re-suspended by adding 80µl 0.5% saponin to obtain suspensions. The obtained suspensions were labeled respectively with T-bet fluorescent monoclonal antibody (0.1 µg), GATA3 fluorescent monoclonal antibody (0.1 µg), RORyT fluorescent monoclonal antibody (0.1 µg), and Foxp3 fluorescent monoclonal antibody (0.1 µg), and incubated in dark place at 4 °C for 1 h (For Foxp3, intraprep permeabilization takes 8 h). The labeled cells were washed three times with 0.5% saponin, centrifuged for 5min at 300g, 4°C to obtain a supernatant and a precipitate. The supernatant was removed, and the precipitate was fixed with 1% paraformaldehyde, and then analyzed with a flow cytometer Accuri-C6 to determine the percentages of the above transcription factors. The antibodies and instruments used in the above steps were all purchased from BD Bioscience.
(4) Results: The results are shown in Fig. 2 and Fig. 3. The CON in Fig. 2 represents a control group, i.e. the lymphocytes of peripheral blood from normal humans not treated with DHA, EPA or AA. The CON in Fig. 3 represents a control group, i.e. the lymphocytes of peripheral blood from patients not treated with DHA, EPA, or AA. In Fig. 2, figures A, B, C, D, and E show percentages of Th1, Th2, Th1/Th2, Th17 and Treg cells in peripheral blood CD4 T-cells of normal subjects, respectively, and figures F, G, H, I and J show percentages of Th1, Th2, Th1/Th2, Th17 and Treg cells in peripheral blood CD4 T-cells of patients, respectively. In Fig. 3, figures A, B, C, and D show percentages of transcription factors T-bet, GATA3, RORyT, and Foxp3 in peripheral blood CD4 T-cells of patients, respectively. As shown in Figs. 2-3, in peripheral blood CD4 T-cells of patients with type 1 diabetes treated with DHA, EPA and AA respectively, the percentages of Th1 and Th17 cells decreased, and the percentages of Th2 and Treg cells increased, and the ratio of Th1/Th2 and the ratio of T17/Treg were rebalanced. The transcription factor T-bet that regulates the expression of Th1 cytokines and the transcription factor RORyT that regulates the expression of Th17 cytokines were inhibited significantly by ω-3PUFAs, while the transcription factors GATA3 and Foxp3 that regulate the expressions of cytokines Th2 and Treg were up-regulated under the action of ω-3PUFAs. These results fully indicate that ω-3 PUFAs have effects of re-balancing Th cell differentiation and significantly inhibiting inflammation caused by the imbalance of the ratios of Th1/Th2 and T17/Treg, and further indicate that the mfat-1 gene carried by the viral particles of the present invention can convert high levels of ω-6 PUFAs in the body into ω-3 PUFAs by using ω-6 PUFAs as a substrate, so that a ratio of ω-6/ω-3 is balanced and tends to 1:1, so as to inhibit autoimmune diseases caused by imbalance of the ratios of Th1/Th2 and T17/Treg, such as type 1 diabetes, multiple sclerosis, rheumatoid arthritis, and systemic lupus erythematosus.

### Example 3: Effect of Lenti-mfat-1 lentiviral particle in the treatment of type 1 diabetes.

### (1) Construction of a mouse model of type 1 diabetes:

Some NOD mice, each weighing 25 g were used. Blood were collected from the mice at a fixed time every day to detect random blood glucose in NOD mice. Mice with blood glucose of more than 11.1 mmol/L for two consecutive weeks were selected. The selected mice was randomly divided into two groups, a control group (Lenti-Con) and a lentiviral treatment group (Lenti-mfat-1), 5 mice in each group.

### (2) Administration method:

In the treatment group, Lenti-mfat-1 lentiviral particles in the form of a concentrate having a virus titer of 10⁸ TU/mL were injected through a tail vein of the mouse in an amount of 10⁹ TU/Kg mouse. In the control group, the pLJM1-CMV-hPGK-EGFP plasmid without mfat-1 gene were injected under the same conditions. Changes in random blood glucose in mice were detected.

### (3) Results and discussions:

### 1) General physiological conditions of NOD mouse after receiving treatment with Lenti-mfat-1 lentiviral particles

The mice after receiving treatment with Lenti-mfat-1 lentiviral particles in the treatment group had normal feeding and drinking situations compared with the mice in the control group. After 2 weeks of treatment, body weights of the mice in the treatment group were higher than that of the control group, but there was no statistical significance.

2) After treatment with the viral particles of example 1 for 9 weeks, a level of polyunsaturated fatty acid in the peripheral blood of the mice is detected. The detailed steps of detecting are as follows: after extracting fatty acids from peripheral blood of mice of the two groups by conventional organic chemical methods, the extracted samples (the extracted fatty acids) were dissolved in heptane, and then dropped into sample bottles for sample loading and testing. The sample was loaded through an automatic sampler and tested by Agilent 7890A. The running time of a single loading was about 1 h. Gas chromatographic conditions: column model: SP2380, 105m^{∗}0.53mm^{∗}0.20µm (Agilent); operating procedures: holding at an initial temperature of 140 °C for 3 min, then rising to 220 °C at a rate of 8 °C/min and holding at this temperature for 12 min; temperature of injector and detector: 260 °C; carrier gas is helium, speed is set to 12 psi. Finally, the type of fatty acid was determined according to peak times of the sample and a standard product (purchased from Sigma, USA), and the percentage of each fatty acid was obtained as the final result. The peak of interest was determined by comparing peak time, peak shape, and peak area percentage with those of the standard product; the sum of all the area percentages of ω-3 and ω-6 polyunsaturated fatty acids was calculated, and the ratio of ω-6/ω-3 was calculated. Column type: SP2380, 105 m^{∗}0.53 mm^{∗}0.20 µm). It was found that the proportion of EPA in mouse receiving Lenti-mfat-1 lentiviral particles increased, indicating the lentiviral particles with mfat-1 gene successfully worked (Table 5).

**Table 5 Changes in polyunsaturated fatty acids in blood of mouse after treatment with viral particles.**

| (%) | Lenti -con | Lenti-mfat-1 |
|---|---|---|
| ω-3 PUFA species | | |
| c18:3 (α-LA) | 0.34 ± 0.12 | 0.36 ± 0.48 |
| C20:5 (EPA) | 0.53 ± 0.09 | 5.75 2.96* |
| C22:5 | 0.59 ± 0.04 | 1.82 ± 1.73 |
| C22:6 (DHA) | 6.26 ± 1.05 | 7.82 ± 5.22 |
| ω-6 PUFA species | | |
| C18:2 | 19.5 ± 1.67 | 16.7 ± 18.3 |
| C18:3 (γ-LA) | NP | NP |
| C20:4 (AA) | 12.2 ± 3.18 | 8.01 ± 4.02 |
| C22:4 | 0.94 ± 0.19 | NP |
| ω-6/ω-3 ratio | 4.24 ± 0.31 | 1.77 ± 1.71 |

Each of ω-3 and ω-6 polyunsaturated fatty acid is expressed as a relative percentage, wherein the peak area of all fatty acids detected by gas chromatography is 100%, and the content of the target fatty acid is calculated by dividing the peak area by the total peak area the target fatty acid. Data of each group was repeated three times, and counted using oneway-ANOVA. Compared with the control group, *P < 0.05, NP: not detected.

### 3) Monitoring of random blood glucose levels, insulin levels, and islet inflammation in two groups of NOD mouse

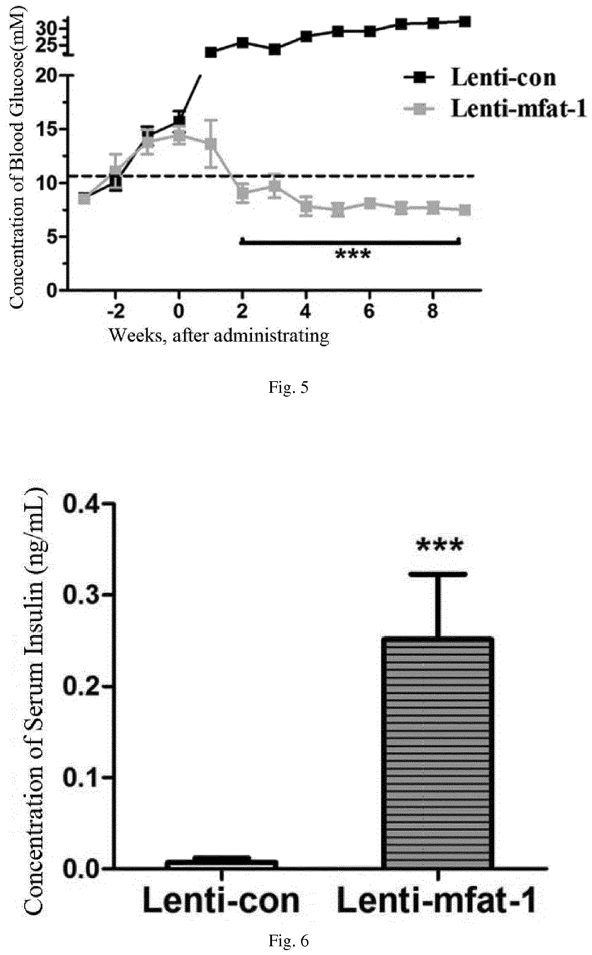

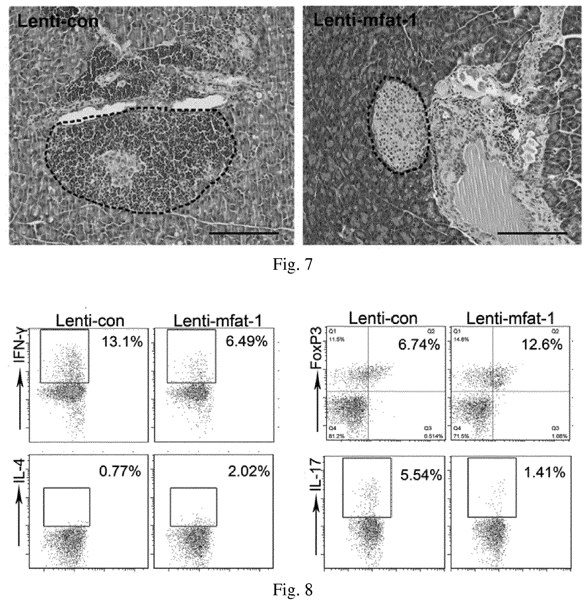

Mouse having random blood glucose of below 11.1 mmol/L for two consecutive weeks was considered to have returned to normal blood glucose. Blood samples were taken from eye sockets of mouse in the Lenti-mfat-1 treatment group and the control group NOD, and then analyzed with an American Roche Accu-Chek blood glucose meter to determine the blood glucose levels. Results were shown in Figs. 4-5. Compared with the control group, the randomized blood glucose of the NOD mouse in the treatment group had been significantly down-regulated at the second week, and had decreased to and maintained at the normal level by the sixth week, reaching 6.3 mmol/L, indicating Lenti-mfat-1 lentiviral particles have a significant therapeutic effect on NOD mouse. After 9 weeks of treatment, 2 ml of blood was taken from eyeballs of the two groups of mouse, left to stand for 5 min, centrifuged at 3500 rpm for 15 min to obtain an upper serum. 200 µl of the upper serum was diluted 100 times with PBS having a pH of 7.4, and then analyzed with a kit purchased from Mercodia to determine the concentration of insulin in the serum. The result was shown in Fig. 6. After 9 weeks of treatment, the pancreas of the two groups of mouse was taken under a stereoscopic microscope, and then routinely embedded in paraffin, sectioned, and further HE stained to observe an infiltration of islet lymphocytes of mouse. The result was shown in Fig. 7.

### 4) Changes in CD4 T lymphocyte differentiation in NOD mouse after treatment with Lenti-mfat-1 lentiviral particles

Spleen lymphocytes (number up to 1×10⁵) in the NOD mouse of the treatment group and the control group were collected and placed to different marked tubes. Labeled fluorescent monoclonal antibodies CD3 (0.1µg) and CD8 (0.1µg) are added into each tube. 1ml paraformaldehyde having a concentration of 4% by weight was then added into each tube, mixed well and incubated at room temperature (20°C-30°C) for 20 min. 1ml intraprep permeabilization reagent, i.e. 0.5% saponin, was added, incubated at room temperature (20°C-30°C) for 30 min, then centrifuged for 5 min at 300 g, 4 °C to obtain a supernatant and a precipitate. The supernatant was removed, and the precipitate was resuspended by adding 80µl 0.5% saponin to obtain suspensions. The obtained suspensions were labeled respectively with IFN-γ fluorescent monoclonal antibody (0.1 µg), IL-17 fluorescent monoclonal antibody (0.1 µg), IL-4 fluorescent monoclonal antibody (0.1 µg), and Foxp3 fluorescent monoclonal antibody (0.1 µg), and incubated in dark place at 4 °C for 1 h (For the sample labeled with Foxp3 fluorescent monoclonal antibody, incubation and rupture takes 8 h). The labeled cells were washed three times with 0.5% saponin, centrifuged for 5 min at 300g, 4°C to obtain a supernatant and a precipitate. The supernatant was removed, and the precipitate was fixed with paraformaldehyde having a concentration of 1% by weight. After 24 h, percentages of Th1, Th2, Th17 and Treg cells were analyzed with a flow cytometer Accuri-C6. The antibodies and instruments used in the above steps were all purchased from BD Bioscience. Results were shown in Fig. 8. In CD4 T cells of NOD mouse treated with Lenti-mfat-1 lentiviral particles, the percentages of Th1 and Th17 cells decreased, and the percentages of Th2 and Treg cells increased, and the ratios of Th1/Th2 and T17/Treg were rebalanced, thereby significantly improving inflammatory environment caused by the imbalance of the ratios of Th1/Th2 and T17/Treg

### Example 4: Mfat-1 transgenic mice completely resist inducers to induce occurrences of MS

(1) Establishment of model: 3 wide type mice (6-8 weeks, two females and one male, purchased from Model Animal Research Center of Nanjing University), and 3 maft-1 transgenic mice (6 weeks, two females and one male, provided by Model Animal Research Center of Nanjing University; the maft-1 transgenic mice had fat-1 gene expressed by pST181 prokaryotic expression vector, and named mfat-1 transgenic mouse because the muscle creatine kinase (MCK) enhancer was designed upstream of the fat-1 gene promoter CMV-β-actin to increase expression efficiency of the fat-1 gene in mammals (mammalianized)) were used. All of them were immunized with MOG by subcutaneous injection of MOG 35-55 (purchased from Sigma) into four points of dorsal areas of immunizing animals at 300 µg/body weight (kg). A second immunization was performed at the same dose six days after the first immunization. Animals survived for 24 days.
(2) Observation index of model:
   Behavioral observation of clinical neuropathy: changes of animal behavioral were observed and recorded every day from the 12th day after establishment of model according to Kerlero scoring method. Scores were recorded according to the following behaviors: 1 point, weakness of tail; 2 points, paralysis of tail; 2.5 points, mild weakness of unilateral hind limbs; 3 points, significantly weakness of unilateral hind limbs; 4 points, paralysis of unilateral hind limbs; 4.5 points, paralysis of unilateral hind limbs accompanied with weakness of contralateral hind limbs or mild weakness of ipsilateral fore limbs; 5 points, paralysis of bilateral hind limbs; 6 points, paralysis of bilateral hind limbs accompanied with paralysis of unilateral fore limbs.
(3) Results: The results were shown in Table 6 below. The normal wild type mouse began to develop mild symptoms of weakness of tail from the 14th day after establishment of model, and by 23th day, all showed near-paralysis of unilateral hind limbs; while under the same induction conditions, mfat-1 transgenic mice showed no obvious symptoms during the whole process. It is indicated that the mfat-1 gene plays significant role in resisting or treating MS in the body.

**Table 6 Behavior test scores of MS in mfat-1 transgenic mice and normal wildtype mice**

| **Groups** | **Behavior test scores of MS** | | | | | | | | | | | |
|---|---|---|---|---|---|---|---|---|---|---|---|---|
| | day 12 | day 13 | day 14 | day 15 | day 16 | day 17 | day 18 | day 19 | day 20 | day 21 | day 22 | day 23 |
| Wild type ♂ | 0 | 0 | 1 | 1 | 1 | 1 | 3.5 | 1 | 3.5 | 3.5 | 3.5 | 3.5 |
| Wild type ♀ | 1 | 1 | 1 | 1 | 1 | 1 | 1 | 3.5 | 3.5 | 3.5 | 3.5 | 3.5 |
| Wild type ♀ | 0 | 0 | 1 | 1 | 1 | 1 | 1 | 3.5 | 1 | 3.5 | 3.5 | 3.5 |
| mfat-1 transgenic mice ♀ | 0 | 0 | 0 | 0 | 0 | 0 | 0 | 0 | 0 | 0 | 0 | 0 |
| mfat-1 transgenic mice ♀ | 0 | 0 | 0 | 0 | 0 | 0 | 0 | 0 | 0 | 0 | 0 | 1 |
| mfat-1 transgenic mice ♂ | 0 | 0 | 0 | 0 | 0 | 0 | 0 | 0 | 0 | 0 | 0 | 1 |

## Claims

1. A viral vector, **characterized in that**, the viral vector is a lentiviral expression plasmid cloned with mfat-1 gene or an adeno-associated viral expression plasmid cloned with mfat-1 gene, and the mfat-1 gene is as shown in SEQ ID No.: 1.

2. The viral vector according to claim 1, **characterized in that**, the lentiviral expression plasmid is pLJM1-CMV-hPGK-EGFP plasmid, pLJM1-CMV-hPGK-mkate2 plasmid, pLenti-CMV-MCS-GFP-SV-puro plasmid, FUGW, pLenti-puro, pLenti-MP2 or pLenti plasmid; and the adeno-associated viral expression plasmid is pEMBL-AAV-D(+)-CMV-eGFP-SV40 plasmid, AAV GFP plasmid, AAV1 plasmid, AAV2 plasmid, rAAV2 plasmid, AAV5 plasmid, AAV8 plasmid, AAV9 plasmid or pAV-FH AAV plasmid.

3. A method for constructing the viral vector according to claim 1 or 2, **characterized in that**, the viral vector is obtained by cloning the mfat-1 gene into a lentiviral expression plasmid or an adeno-associated viral expression plasmid.

4. The method according to claim 3, **characterized in that**, the method comprises steps of:
(1) designing primers by using mfat-1 gene sequence as a template, and carrying out PCR amplification of the mfat-1 gene sequence with the primers, to obtain a PCR amplification product which is a mfat-1 gene sequence with NheI and EcoRI restriction sites at both ends; wherein the primers are as follows:
mfat-1-F: 5'-TATTAAGCTAGCATGGTCGCCCACAGCA-3';
mfat-1-R: 5'-CAACCGGAATTCTCATCACTTGGCCT-3';
(2) electrophoresing the PCR amplification product obtained in the step (1) to obtain a gel, then cutting, recovering and purifying the gel to obtain a purified PCR amplification product, digesting the purified PCR amplification product with restriction enzymes NheI and EcoR to obtain a DNA fragment, and digesting an empty lentiviral expression plasmid or adeno-associated viral expression plasmid with restriction enzymes NheI and EcoR to obtain a digested empty shuttle plasmid;
(3) ligating the DNA fragment obtained in step (2) to the digested empty shuttle plasmid to obtain the viral vector.

5. The method according to claim 3 or 4, **characterized in that**, a reaction system of the PCR amplification in the step (1) comprises:
5×PrimeSTAR® Buffer (Mg2+ plus), 10 µl;
dNTP Mixture, each dNTP 2.5 mM, 4 µl;
template DNA, 20-200ng;
upstream primer mfat-1-F, 10 µM, 1 µl;
downstream primer mfat-1-R, 10 µM, 1 µl;
PrimeSTAR® HS DNA Polymerase, 2.5 U/µl, 0.5 µl;
adding sterile ultrapure water to 50µL.

6. The method according to claim 3 or 4, **characterized in that**, a reaction procedure of the PCR amplification in the step (1) is as follows: denaturation at 98 °C for 5 min; and holding at 98 °C for 10 s, and holding at 60 °C for 15 s, holding at 72 °C for 2 min, a total of 30 cycles; finally extension at 72 °C for 10 min.

7. A recombinant viral vector, **characterized in that**, the recombinant viral vector is constructed by the viral vector of claim 1 or 2.

8. A viral particle, **characterized in that**, the viral particle is obtained by transforming the viral vector according to claim 1 or 2 into a cell.

9. The viral particle according to claim 8, **characterized in that**, the viral particle is administered by intravenous injection.

10. Use of the viral vector of claim 1 or 2 or the recombinant viral vector of claim 7 or the viral particle of claim 8 in the manufacture of a medicament for treating diabetes or autoimmune related diseases.

11. The use according to claim 10, **characterized in that**, the diabetes is autoimmune type 1 diabetes.

12. The use according to claim 10, **characterized in that**, the autoimmune related diseases are autoimmune diseases induced by imbalance of Th cell differentiation and imbalance of its secreted cytokines.

13. The use according to claim 12, **characterized in that**, the autoimmune disease is selected from type 1 diabetes, multiple sclerosis, rheumatoid arthritis and systemic lupus erythematosus.

14. A medicament, **characterized by** comprising the viral vector of claim 1 or 2 or the recombinant viral vector of claim 7 or the viral particle of claim 8, and a pharmaceutically acceptable carrier thereof.

15. The medicament according to claim 14, **characterized in that**, the medicament is prepared into a clinically acceptable dosage form with a conventional process by mixing the viral vector of claim 1 or 2 or the recombinant viral vector of claim 7 or the viral particle of claim 8 with conventional excipients.
